# EUROPÄISCHE PATENTSCHRIFT

(11) **EP 3 210 071 B1**
(45) Veröffentlichungstag und Bekanntmachung des Hinweises auf die Patenterteilung: **08.11.2023**
(21) Anmeldenummer: 15774505.0
(22) Anmeldetag: 15.09.2015
(51) Int. Cl.: G02C 13/00, A61B 3/11, A61B 3/113

(54) **VORRICHTUNG UND VERFAHREN ZUM BESTIMMEN VON OPTISCHEN PARAMETERN**
APPARATUS AND METHOD FOR DETERMINING OPTICAL PARAMETERS
DISPOSITIF ET PROCÉDÉ DE DÉTERMINATION DE PARAMÈTRES OPTIQUES

(30) Priorität: 23.10.2014 DE 102014015671
(43) Veröffentlichungstag der Anmeldung: 30.08.2017
(73) Patentinhaber: Rodenstock GmbH, 80687 München (DE)
(72) Erfinder: SEITZ, Peter, 80999 München (DE); TIEMANN, Markus, 81539 München (DE); ESSER, Gregor, 80686 München (DE); MÜLLER, Werner, 75443 Ötisheim (DE)
(74) Vertreter: Müller-Boré & Partner Patentanwälte PartG mbB
(86) Internationale Anmeldenummer: PCT/EP2015/001844
(87) Internationale Veröffentlichungsnummer: WO 2016/062363

(56) Entgegenhaltungen:
- DE-A1-102005 003 699
- DE-A1-102005 003 699
- DE-A1-102010 007 922
- DE-A1-102010 007 922
- MUFF B: "DIE BRILLE AUS DEM COMPUTER", TECHNISCHE RUNDSCHAU, HALLWAG AG, CH, Bd. 80, Nr. 45, 4. November 1988 (1988-11-04), Seite 52/53, XP000098546, ISSN: 1023-0823
- MUFF B: "DIE BRILLE AUS DEM COMPUTER", TECHNISCHE RUNDSCHAU, HALLWAG, BERN; CH, DE, vol. 80, no. 45, 4 November 1988 (1988-11-04), page 52/53, XP000098546, ISSN: 1023-0823

## Beschreibung

Die vorliegende Erfindung betrifft eine Vorrichtung zum Bestimmen von optischen Parametern eines Benutzers, ein Verfahren zum Bestimmen von optischen Parametern eines Benutzers sowie ein Computerprogrammprodukt zum Durchführen des Verfahrens.

Durch die Einführung von individuell optimierten Brillengläsern ist es möglich, auf die Ansprüche von Personen mit Sehfehlern einzugehen und beispielsweise Brillengläser mit individuell optimierten Sehbereichen bereitzustellen. Individuell angepasste Brillengläser ermöglichen eine optimale Korrektur von optischen Sehfehlern eines Benutzers der Brillengläser. Eine individuelle Berechnung und Anpassung von Brillengläsern ist auch für Sportbrillen möglich, welche sich durch große Durchbiegungen, Fassungsscheiben- und Vorneigungswinkel auszeichnen.

Um die optischen Vorteile von individuellen Brillengläsern, insbesondere von individuell angepassten Gleitsichtgläsern, vollständig auszuschöpfen, ist es notwendig, diese Brillengläser in Kenntnis der Gebrauchsstellung des Benutzers zu berechnen und herzustellen und gemäß der zur Berechnung und Herstellung verwendeten Gebrauchsstellung zu tragen. Die Gebrauchsstellung ist von einer Vielzahl von Parametern abhängig, beispielsweise von der Pupillendistanz des Benutzers, dem Fassungsscheibenwinkel, der Brillenglasvorneigung, der Brillenfassung, dem Hornhautscheitelabstand des Systems von Brille und Auge und der Einschleifhöhe der Brillengläser. Diese und weitere Parameter, welche zur Beschreibung der Gebrauchsstellung herangezogen werden können, bzw. notwendig sind, sind in einschlägigen Normen, wie beispielsweise der DIN EN ISO 1366, der DIN 58 208, der DIN EN ISO 8624 und der DIN 5340 enthalten und können diesen entnommen werden. Ferner ist es notwendig, dass die Brillengläser entsprechend den optischen Parametern, welche zur Herstellung verwendet wurden, in einer Brillenfassung angeordnet bzw. zentriert werden, so dass die Brillengläser tatsächlich entsprechend den optischen Parametern in Gebrauchsstellung getragen werden.

Zur Bestimmung der Lage eines Brillenglases vor dem Auge müssen mehrere Parameter bestimmt werden. Dadurch können einerseits die zum Einschleifen und Einsetzen in die Fassung benötigten Informationen erhalten werden, andererseits können damit Optimierungen im Brillenglas selbst vorgenommen werden, um es auf die Trageposition in Gebrauchsstellung anzupassen.

Zur Bestimmung solcher Parameter ist z.B. aus der DE 10 2005 003 699 A1 eine Vorrichtung mit zwei Bildaufnahmevorrichtungen bekannt, die aus unterschiedlichen Aufnahmerichtungen jeweils ein Bild von einem Benutzer mit Brille aufnimmt, aus denen sie z.B. dreidimensionale Benutzerdaten errechnet.

Dokument DE 10 2010 007 922 A1 betrifft eine Vorrichtung und ein Verfahren zum Ermitteln eines Pupillenabstandes. Gemäß einer ersten Ausführungsform wird der Pupillenabstand stereoskopisch ermittelt, d.h. anhand zweier Aufnahmen des Kopfes eines Probanden aus unterschiedlichen Aufnahmerichtungen. Gemäß einer anderen Ausführungsform wird der Pupillenabstand anhand einer Bildaufnahme ermittelt, wobei auf den Kopf des Probanden zusätzlich ein Streifenmuster projiziert wird. Die beiden Berechnungsverfahren können kombiniert werden, wobei das Streifenmuster zur Bestimmung des Konvergenzwinkels zwischen den beiden Aufnahmerichtungen verwendet werden kann.

Der Artikel "Die Brille aus dem Computer" von B. Muff betrifft die Berechnung optischer Parameter mit Hilfe einer Bildverarbeitungssoftware aus den Bildern zweier Videokameras.

Andere Vorrichtungen arbeiten mit zweidimensionalen Berechnungsverfahren, die anhand mehrerer Bilder die gewünschten Parameter ermitteln. Zudem gibt es manuelle Bestimmungsmöglichkeiten, wie z.B. ein Pupillometer und ein Pupillenabstandslineal.

Bei allen diesen Messverfahren besteht eine Abhängigkeit von der messenden Person und der Durchführung der Messung. So kann z.B. anhand einer zweidimensionalen Aufnahme der individuelle Hornhautscheitelabstand bei der Bestimmung der Pupillendistanz nicht ermittelt werden, wodurch sich eine systematische, von Proband und Fassung abhängige Abweichung der Pupillendistanz ergibt.

Bei Verwendung eines stereoskopischen Kamerasystems zur Ermittlung der optischen Parameter eines Benutzers im dreidimensionalen Raum tritt ein Korrespondenzproblem auf. Das Korrespondenzproblem betrifft ein Identifizieren von zueinander korrespondierenden Punkten in zwei aus unterschiedlichen Perspektiven aufgenommenen Aufnahmen. Erst nachdem zueinander korrespondierende Punkte in beiden Aufnahmen ermittelt worden sind, kann eine 3D-Rekonstruktion der Aufnahmepunkte erfolgen.

In der Praxis werden die korrespondierenden Punkte durch eine manuelle Auswertung der Aufnahmen ermittelt. Diese manuelle Auswertung erfordert einen erheblichen Zeitaufwand und ist durch die Nutzerabhängigkeit eine potentielle Fehlerquelle für die 3D-Rekonstruktion.

Der Erfindung liegt die Aufgabe zugrunde, eine verbesserte Möglichkeit zum Ermitteln von optischen Parametern eines Benutzers bereitzustellen.

Diese Aufgabe wird durch die Gegenstände der unabhängigen Ansprüche gelöst.

Ein erster Aspekt betrifft eine Vorrichtung gemäß unabhängigem Anspruch 1.

Die Projektionseinrichtung erzeugt die Lichtprojektion. Die Projektionseinrichtung kann als ein Strahler ausgebildet sein, der elektromagnetische Strahlung erzeugt. Die Projektionseinrichtung kann zum Beispiel als LED oder als Laser ausgebildet sein. Die Vorrichtung weist zumindest eine Projektionseinrichtung auf, kann allerdings zusätzlich eine oder mehrere weitere Projektionseinrichtungen aufweisen, die einen erweiterten Teilbereich des Kopfes des Benutzers und/oder der Brille des Benutzers bestrahlen.

Der dabei beleuchtete, also markierte, Teilbereich des Kopfes des Benutzers und/oder der Brille des Benutzers betrifft einen Teilbereich des Systems "Kopf mit Brille", also des Kopfes des Benutzers und der daran in Gebrauchsstellung angeordneten Brille des Benutzers. Die Gebrauchsstellung ist zum Beispiel in den eingangs benannten Normen definiert. Mit der Lichtprojektion kann entweder ein Teilbereich des Kopfes, ein Teilbereich der Brille oder bevorzugt ein Teilbereich markiert werden, der sowohl Teile des Kopfes als auch Teile der Brille aufweist. Markierte Punkte im Teilbereich des Kopfes können zum Beispiel eine oder die beiden Pupillen sein, insbesondere die Pupillenmittelpunkte, sowie die Nasenwurzel des Benutzers, sofern nicht von der Brillenfassung bedeckt. Punkte des markierten Teilbereichs der Brille können insbesondere Punkte auf der Brillenfassung sein, zum Beispiel temporal und/oder nasal angeordnete innere und/oder äußere Fassungsbegrenzungspunkte, sowie oberhalb und/oder unterhalb der Pupillen angeordnete innere und/oder äußere Fassungsbegrenzungspunkte. Mit der Lichtprojektion können mehrere Punkte des Teilbereichs markiert werden.

Die Markierung erfolgt dabei bevorzugt mit einer Lichtwellenlänge, die in den Bilddaten schnell und sicher erkannt werden kann, z.B. automatisch über eine Computer gesteuerte Auswertung.

Als Bildaufnahmeeinrichtung kann zum Beispiel eine digitale Kamera verwendet werden. Die Bildaufnahmeeinrichtung kann eine digitale Kamera und zumindest ein optisches Umlenkelement bzw. einen Umlenkspiegel umfassen, wobei die Bilddaten des Teilbereichs mit der Kamera mittels des Umlenkspiegels bzw. Umlenkelements aufgezeichnet und/oder erzeugt werden.

Die Bildaufnahmeeinrichtung erzeugt Bilddaten, was durch das Aufnehmen eines Bildes erfolgen kann. Die Bilddaten können somit digitale Daten einer Aufnahme darstellen. Dabei enthält die so erzeugte Aufnahme zumindest den von der Lichtprojektion markierten Teilbereich des Kopfes und/oder der Brille des Benutzers. Die Bilddaten umfassen vorzugsweise eine Aufnahme von beiden Augen des Benutzers, insbesondere von den beiden Pupillen des Benutzers sowie der Fassung der Brille des Benutzers. Der markierte Teilbereich betrifft zumindest einzelne Punkte in dieser Aufnahme und somit zumindest einzelne Punkte in diesen Bilddaten.

Die Vorrichtung kann ein stereoskopisches Kamerasystem aufweisen, also zum Beispiel zwei Bildaufnahmeeinrichtungen aufweisen, die aus zwei unterschiedlichen Perspektiven Bilddaten des markierten Teilbereichs erzeugen. Alternativ kann die Vorrichtung lediglich eine verschwenkbare Bildaufnahmeeinrichtung aufweisen, die aus zwei unterschiedlichen Perspektiven Bilddaten von dem markierten Teilbereich erzeugt.

Die Bilddaten umfassen zumindest den markierten Teilbereich des Systems Kopf mit Brille, können allerdings weitere Teile des Kopfes und/oder der Brille umfassen.

Die Datenverarbeitungseinrichtung kann als Computer ausgebildet sein und/oder einen Mikroprozessor aufweisen. Die Benutzerdatenbestimmungseinrichtung und die Parameterbestimmungseinrichtung können unabhängig voneinander arbeiten. Die Datenverarbeitungseinrichtung kann derart ausgelegt sein, dass die Benutzerdatenbestimmungseinrichtung und die Parameterbestimmungseinrichtung mittels eines gemeinsamen Mikroprozessors betrieben werden. In anderen Worten ist die Datenverarbeitungseinrichtung derart ausgelegt, dass zumindest ein Mikroprozessor sowohl die Aufgabe(n) der Benutzerdatenbestimmungseinrichtung als auch der Parameterbestimmungseinrichtung ausführt.

Die Bildaufnahmeeinrichtung kann derart ausgelegt und angeordnet sein, dass in den erzeugten Bilddaten zumindest eine Pupille des Benutzers und ein Pupillenfassungsrand und/oder ein Brillenglasrand abgebildet ist, wobei in den erzeugten Bilddaten die zumindest eine Pupille des Benutzers von dem Brillenfassungsrand und/oder dem Brillenglasrand umgrenzt ist.

Die Benutzerdatenbestimmungseinrichtung erzeugt Benutzerdaten. Die Benutzerdaten enthalten Ortsinformationen für einige Punkte des markierten Teilbereichs. Die Benutzerdaten werden aus den Bilddaten generiert, die die Markierung durch die Lichtprojektion beinhalten. Dazu ist die Bildaufnahmeeinrichtung derart ausgelegt, dass sie die Lichtwellenlänge der Lichtprojektion detektieren kann. Die Bildaufnahmeeinrichtung ist also sensitiv für den Lichtwellenbereich der Lichtprojektion ausgebildet. Die Benutzerdaten können Ortsinformationen im dreidimensionalen Raum von einzelnen Punkten des markierten Teilbereichs enthalten, und/oder eine vollständige 3D-Rekonstruktion der Aufnahme ermöglichen. Eine 3D-Rekonstruktion kann mathematisch zum Beispiel aus zwei Aufnahmen mittels Epipolargeometrie erfolgen. Die Benutzerdaten können Ortsinformationen für zumindest einen der folgenden Punkte enthalten:
- Einen Schnittpunkt einer im Bezugssystem des Benutzers horizontalen Ebene mit den Brillenglasrändern und/oder den Brillenfassungsrändern der Brille, wobei die horizontale Ebene des Benutzers beide Pupillen des Benutzers schneidet und parallel zu einer vorbestimmten Nullblicklinie des Benutzers verläuft;
- Schnittpunkt einer im Bezugssystem des Benutzers vertikalen Ebene mit den Brillenglasrändern und/oder den Brillenfassungsrändern der Brille, wobei die vertikale Ebene des Benutzers senkrecht zur horizontalen Ebene des Benutzers und parallel zu der vorbestimmten Nullblicklinie des Benutzers verläuft und eine Pupille des Benutzers schneidet;
- zumindest einen Pupillenmittelpunkt;
- Begrenzungen zumindest eines Brillenglases des Benutzers nach einer Bemaßung im Kastenmaß;
- Brillenmittelpunkt der Brillenfassung der Brille.

Unter einer Bemaßung im Kastenmaß wird im Sinne dieser Erfindung das Maßsystem verstanden, wie es in einschlägigen Normen, beispielsweise in der DIN EN ISO 8624 und/oder der DIN EN ISO 1366 DIN und/oder der DIN 58208 und/oder der DIN 5340 beschrieben wird. Ferner wird hinsichtlich des Kastenmaßes und weiterer verwendeter herkömmlicher Begriffe und Parameter auf das Buch "Die Optik des Auges und der Sehhilfen" von Dr. Roland Enders, 1995 Optische Fachveröffentlichung GmbH, Heidelberg, sowie das Buch "Optik und Technik der Brille" von Heinz Diepes und Ralf Blendowski, 2002 Verlag Optische Fachveröffentlichungen GmbH, Heidelberg, verwiesen. Die Normen sowie das genannte Buch stellen für die Begriffsdefinitionen insoweit einen integralen Offenbarungsbestandteil der vorliegenden Anmeldung dar.

Die Begrenzungen nach einer Bemaßung im Kastenmaß umfassen beispielsweise Fassungspunkte für ein Auge oder beide Augen, welche am weitesten außen bzw. innen und/oder oben bzw. unten liegen. Diese Fassungspunkte werden herkömmlicherweise anhand von Tangenten an die Brillenfassung bzw. den jeweiligen Augen zugeordneten Bereichen der Brillenfassung bestimmt (vgl. z.B. DIN 58 208; Bild 3).

Die Nullblickrichtung im Sinne dieser Erfindung ist eine Blickrichtung geradeaus bei parallelen Fixierlinien. In anderen Worten handelt es sich um eine Blickrichtung, welche durch eine Stellung des Auges relativ zum Kopf des Benutzers definiert ist, wobei die Augen ein Fixierobjekt anblicken, das sich in Augenhöhe befindet und an einem unendlich fernen Punkt angeordnet ist. Als Fixierobjekt kann z.B. die Bildaufnahmeeinrichtung verwendet werden. Da das reale Fixierobjekt nicht im Unendlichen angeordnet sein kann, kann in der Praxis aus dem Abstand der Augen zum Fixierobjekt unter Zuhilfenahme eines Augenmodells die Blickrichtung korrigiert werden, so dass diese der Nullblickrichtung entspricht. Folglich ist die Nullblickrichtung im Sinne dieser Erfindung lediglich durch die Stellung der Augen relativ zum Kopf des Benutzers bestimmt. Befindet sich der Kopf des Benutzers in einer normalen aufrechten Haltung, so entspricht die Nullblickrichtung im Wesentlichen der Horizontalrichtung im Bezugssystem der Erde. Die Nullblickrichtung kann aber zu der Horizontalrichtung im Bezugssystem der Erde gekippt sein, falls beispielsweise der Benutzer seinen Kopf, ohne weitere Bewegung der Augen, nach vorne oder zur Seite neigt. Analog wird durch die Nullblickrichtung beider Augen eine Ebene aufgespannt, welche im Bezugssystem der Erde im Wesentlichen parallel zur Horizontalebene ist. Die Ebene, welche durch die beiden Nullblickrichtungen der beiden Augen aufgespannt wird, kann ebenfalls zu der Horizontalebene im Bezugssystem der Erde geneigt sein, falls beispielsweise der Benutzer den Kopf vorne oder zur Seite neigt.

Die horizontale Ebene des Benutzers kann einer ersten Ebene entsprechen. Die vertikale Ebene des Benutzers kann einer zweiten Ebene entsprechen, welche senkrecht zu der ersten Ebene ist. Beispielsweise kann die horizontale Ebene im Bezugssystem des Benutzers parallel zu einer horizontalen Ebene im Bezugssystem der Erde angeordnet sein und lediglich durch den Mittelpunkt einer Pupille verlaufen. Dies ist insbesondere dann der Fall, falls die beiden Augen des Benutzers beispielsweise in unterschiedlicher Höhe (im Bezugssystem der Erde) angeordnet sind.

Die Parameterbestimmungseinrichtung bestimmt aus den Benutzerdaten die gesuchten optischen Parameter des Benutzers. Die optischen Parameter des Benutzers können zumindest einen der folgenden Werte umfassen:
- Pupillendistanz;
- monokularer Pupillenabstand;
- Hornhautscheitelabstand nach Bezugspunktforderung und/oder nach Augendrehpunktforderung;
- monokularer Zentrierpunktabstand;
- Zentrierpunktkoordinaten;
- Scheibenabstand;
- Dezentration des Zentrierpunktes;
- Scheibenhöhe und -breite;
- Scheibenmittenabstand;
- Brillenglasvorneigung;
- Fassungsscheibenwinkel;
- Einschleifhöhe.

Ferner umfassen die optischen Parameter weiterhin vorzugsweise einen Augendrehpunkt eines Auges und/oder Parameter, anhand welchem ein dynamisches Sehverhalten eines Benutzers bestimmt werden kann, wie beispielsweise Konvergenz einer Augenstellung und/oder Blickauslenkung.

Die Pupillendistanz entspricht im Wesentlichen dem Abstand der Pupillenmitten.

Die optischen Parameter umfassen besonders bevorzugt physiologische und anatomische Parameter eines Brillenträgers, fassungsspezifische Eigenschaften sowie Merkmale eines Systems Brille-Auge des Benutzers, welches beispielsweise in der DIN 58208 beschrieben ist. Die Merkmale des Systems Brille-Auge des Benutzers können beispielsweise zur Berechnung von Brillengläsern und zur genauen Zentrierung von Brillengläsern verwendet werden, Zentrierdaten gemäß der zitierten Normen exakt bzgl. einer Scheiben- bzw. einer Fassungsebene bestimmt werden. Die Scheibenebene ist hierbei die Ebene durch eine horizontale und vertikale (im Bezugssystem der Erde) Mittellinie im rechten bzw. linken Kastensystem in der Brillenfassung. Die Fassungsebene ist die Ebene durch zueinander vertikale Mittellinien der die rechte und linke Scheibenebene der Brillenfassung festlegenden Kastensysteme.

Durch die Lichtprojektion der Projektionseinrichtung werden Punkte des Teilbereiches so markiert, dass eine Identifizierung bestimmter Positionen in den Bilddaten zumindest vereinfacht wird. Insbesondere kann durch die Markierung eine automatisierte bzw. halbautomatisierte Identifizierung bestimmter Positionen in den Bilddaten ermöglicht werden. So können zum Beispiel zueinander korrespondierende Punkte in Aufnahmen eines stereoskopischen Kamerasystems identifiziert werden. Dadurch kann z.B. das für eine 3D-Rekonstruktion relevante Korrespondenzproblem gelöst werden. Dadurch, dass dieselben Punkte, also zueinander korrespondierende Punkte, in beiden Aufnahmen durch dieselbe Markierung (nämlich durch die Lichtprojektion) markiert sind, können korrespondierende Punkte einfach und schnell identifiziert werden. Die Datenverarbeitungseinrichtung kann dazu ausgebildet sein, entweder automatisch bestimmte markierte Punkte in den Bilddaten zu erkennen und weiter zu verwenden, oder einem Bediener wie zum Beispiel einem Optiker ausgewählte Punkte vorzuschlagen, die dieser einfach und schnell bestätigen oder ablehnen kann.

Die Vorrichtung vereinfacht somit das Lösen des Korrespondenzproblems und ermöglicht eine zumindest teilautomatisierte und/oder computergesteuerte Identifizierung von einzelnen Punkten in den Bilddaten. Dadurch wird die Gefahr einer möglicherweise fehlerhaften 3D-Rekonstruktion durch den Bediener reduziert.

Weiterhin ermöglicht die Vorrichtung eine einfache Bestimmung der optischen Parameter, insbesondere ohne weitere körperliche Markierungen an der Fassung der Brille und/oder am Benutzer.

In einer Ausführungsform ist die Projektionseinrichtung so ausgelegt und angeordnet, dass durch die Lichtprojektion in den Bilddaten gezielt einzelne Punkte auf dem Kopf und/oder der Brille des Benutzers markiert werden. Dabei ist die Projektionseinrichtung derart ausgebildet, dass die Lichtprojektion gezielt auf vorbestimmte Punkte des Systems Kopf mit Brille gerichtet werden kann, so dass diese gezielt markiert werden können. Der Teilbereich kann mehrere solcher einzelner Punkte aufweisen, die aufgrund der Markierung in den Bilddaten einfach identifiziert werden können, insbesondere computergesteuert oder computergestützt.

In einer Weiterbildung dieser Ausführungsform ist die Projektionseinrichtung so ausgelegt und angeordnet, dass in den Bilddaten zumindest einer der folgenden Benutzerpunkte gezielt markiert ist:
- ein Pupillenmittelpunkt,
- ein äußerer temporaler Fassungspunkt,
- ein innerer nasaler Fassungspunkt,
- ein innerer Fassungspunkt oberhalb der Pupille und/oder
- ein innerer Fassungspunkt unterhalb der Pupille.

Die Begriffe oberhalb und unterhalb beziehen sich dabei auf das Bezugssystem des Benutzers, wobei ,oberhalb` im Wesentlichen vertikal oberhalb bedeutet, und 'unterhalb' im Wesentlichen vertikal unterhalb. Hierbei bezieht sich der Begriff vertikal auf das Bezugssystem des Benutzers. Die Begriffe nasal und temporal beziehen sich auf von der Pupille im Wesentlichen horizontal beabstandete Punkte auf der Fassung. Aus den oben angegebenen Benutzerpunkten, insbesondere aus allen zehn der genannten Benutzerpunkte (für jedes Auge jeweils die fünf genannten Benutzerpunkte) lassen sich besonders günstig optische Parameter des Benutzers ermitteln, die für den Optiker wichtig sein können. Somit werden bereits vorzugsweise mit der Lichtprojektion günstige Benutzerpunkte im Teilbereich des Kopfes und/oder der Brille markiert, die von der Benutzerdatenbestimmungseinrichtung in Benutzerdaten mit dreidimensionalen Ortsinformationen umgerechnet werden. Die markierten Punkte können einige Benutzerpunkte oder bereits alle Punkte (inklusive der Benutzerpunkte) enthalten, über die Ortsinformationen im dreidimensionalen Raum bestimmt werden.

Dabei können bei randlosen Fassungen alternativ zu den genannten Fassungspunkten entsprechende Brillenrandpunkte markiert werden.

Gemäß einer Ausführungsform ist die Projektionseinrichtung so ausgelegt und angeordnet, dass die Lichtprojektion in den Bilddaten zumindest teilweise die Form zumindest einer Linie, zumindest einer Linienkreuzung und/oder zumindest eines Punktes aufweist. Dünne Markierungen wie zum Beispiel eine Linie ermöglichen eine besonders einfache Identifizierung von Punkten in den Bilddaten, zum Beispiel als Schnittpunkt der Linie mit der Fassung der Brille, dem Pupillenmittelpunkt, einem inneren und/oder äußeren Fassungsrand, einem Brillenrand, etc. Die Punkte können mit einer Linie, mit einer Linienkreuzung und/oder mittels eines Punktes der Projektionseinrichtung markiert sein. Die Lichtprojektion kann zum Beispiel eine von einem optischen Mittelpunkt der Projektionseinrichtung ausgehende Projektionsebene aufweisen, die den Teilbereich im Wesentlichen in Form einer Linie markiert. Diese Linie passt sich den optischen Gegebenheiten des Benutzers und/oder der Brillenfassung an und kann deswegen in den Bilddaten leichte Biegungen aufweisen, und/oder Unterbrechungen, zum Beispiel beim Sprung vom Gesicht des Benutzers auf einen Fassungsrand. Somit sind hierbei die Begriffe ,Linie', ,Linienkreuzung` und/oder 'Punkt' nicht mathematisch exakt zu verstehen, sondern insofern, dass die von der Lichtprojektion markierten Punkte im Wesentlichen auf einer Linie liegen, oder im Wesentlichen auf einem Punkt. Durch eine derartige Ausbildung der Projektionseinrichtung lassen sich einzelne Punkte in den Bilddaten besonders einfach identifizieren.

Die Lichtprojektion kann derart ausgebildet sein, dass sie auf dem System Kopf mit Brille in zumindest einer Dimension eine maximale Ausdehnung von 2 mm aufweist, bevorzugt von maximal 1 mm. Diese maximale Ausdehnung kann sich z.B. auf eine maximale Linienbreite und/oder einen maximalen Punktdurchmesser auf dem System Kopf mit Brille beziehen.

Gemäß einer Ausführungsform ist die Projektionseinrichtung relativ zur Bildaufnahmeeinrichtung kalibriert und die Benutzerdatenbestimmungseinrichtung benutzt Information über diese Kalibrierung zur Bestimmung der Benutzerdaten. Hierbei umfasst die Kalibrierung Informationen über die Position und die Projektionsrichtung der Projektionseinrichtung relativ zur Position und zum Richtungsvektor der optischen Achse der Bildaufnahmeeinrichtung. Der optische Mittelpunkt der Projektionseinrichtung kann zum Beispiel in einem wohl definierten Abstand zum optischen Mittelpunkt der Bildaufnahmeeinrichtung angeordnet sein. Weiterhin können der Richtungsvektor der Projektionsrichtung und der Richtungsvektor der optischen Achse der Bildaufnahmeeinrichtung in dem Bezugssystem der Vorrichtung vorbekannt sein. Diese vorbekannten Informationen über Richtung und Position werden als Kalibrierung bezeichnet. Die Kalibrierung der Projektionseinrichtung relativ zur Bildaufnahmeeinrichtung kann fixiert sein. Alternativ kann eine der beiden Einrichtungen auch variabel verstellbar sein, wobei in dieser Ausführungsform die Relativposition zueinander, also die Kalibrierung der beiden Einrichtungen gemessen und bestimmt werden kann, und anschließend in der Benutzerdatenbestimmungseinrichtung hinterlegt werden kann.

Die Benutzerdatenbestimmungseinrichtung benutzt Informationen über die Kalibrierung zur Bestimmung der Benutzerdaten. Weist die Lichtprojektion zum Beispiel die Form einer Linie auf und ist parallel zu den Zeilen der Bilddaten angeordnet, kann der Abstand der Vorrichtung vom Benutzer mittels Triangulation ermittelt werden, abhängig von der Zeilenhöhe der projizierten Linie in den Bilddaten. Sind Informationen über die Kalibrierung in der Benutzerdatenbestimmungseinrichtung hinterlegt, so kann die Vorrichtung mit einer einzigen Aufnahme, also einem einzigen Satz Bilddaten, die nötigen optischen Parameter des Benutzers ermitteln. Die Benutzerdaten können hierbei insbesondere im Bezugssystem der kalibrierten Vorrichtung als dreidimensionale Ortsinformationen ermittelt werden.

Gemäß einer Ausführungsform weist die Projektionseinrichtung eine einstellbare Projektionsrichtung auf. So kann die Projektionseinrichtung zusammen mit zumindest Teilen der Vorrichtung derart beweglich ausgebildet sein, dass die Projektionsrichtung eingestellt werden kann. So kann zum Beispiel, wenn die Lichtprojektion die Form einer Linie aufweist, diese Linie bei der Aufnahme der Bilddaten durch die beiden Pupillenmittelpunkte des Benutzers gelegt werden. Zusätzlich zur Projektionsrichtung kann auch die Position der Projektionseinrichtung einstellbar sein. Dies ermöglicht eine gezielte Markierung von gezielt ausgewählten Punkte im markierten Teilbereich des Kopfes des Benutzers und/oder der Brille des Benutzers, die besonders günstig sind für die Ermittlung der optischen Parameter (z.B. einige oder alle der oben genannten Benutzerpunkte). Die Lichtprojektion kann zusätzlich oder alternativ so eingestellt werden, dass zumindest ein Teil der Lichtprojektion im Wesentlichen vertikal (im Bezugssystem des Benutzers) durch zumindest eine Pupille des Benutzers verläuft, so dass die Lichtprojektion die Brillenfassung vertikal schneidet.

Gemäß einer Ausführungsform enthält die von der Projektionseinrichtung bereit gestellte Lichtprojektion zumindest eine Projektionsebene, die auf dem beleuchteten Teilbereich des Kopfes und/oder der Brille des Benutzers zumindest teilweise eine Linie projiziert. Schnittpunkte der Linie mit der Fassung der Brille, einem Brillenrand und/oder dem Pupillenmittelpunkt können hierbei einzelne Punkte in den Bilddaten markieren.

In einer Weiterbildung dieser Ausführungsform ist die Projektionseinrichtung relativ zur Bildaufnahmeeinrichtung so kalibriert, dass sich die optische Achse der Bildaufnahmeeinrichtung und die Projektionsebene unter einem vorbekannten Schnittwinkel schneiden. Im Rahmen dieser Anmeldung ist die optische Achse der Bildaufnahmeeinrichtung nicht zwingenderweise die optische Achse einer Kamera selber, sondern die optische Achse, die, zum Beispiel nach Umlenkung an einem oder mehreren Spiegeln, auf das System Kopf mit Brille des Benutzers trifft. Ähnlich bezeichnet die Projektionsebene diejenige Ebene der Lichtprojektion, die auf das System Kopf mit Brille des Benutzers trifft. Auch die Projektionseben und/oder die Projektionsrichtung der Lichtprojektion kann, nachdem sie die Lichtquelle wie zum Beispiel LED oder Laser verlässt, durch ein oder mehrere optische Bauelemente wie Spiegel, Prisma, Strahlaufweiter, etc. verändert werden. Informationen über diese Kalibrierung ermöglicht ein Berechnen von Benutzerdaten durch die Benutzerdatenbestimmungseinrichtung.

In einer Weiterbildung dieser Ausführungsform beträgt der vorbekannte Schnittwinkel zumindest 10° und maximal 70°. Vorbekannte Schnittwinkel in dieser Größe eignen sich besonders gut als vorgegebene Kalibrierung der Vorrichtung. Bevorzugt beträgt der vorbekannte Schnittwinkel zumindest 20°, besonders bevorzugt zumindest 30°. Weiterhin bevorzugt beträgt der vorbekannte Schnittwinkel maximal 60°. In einer besonders bevorzugten Ausführungsform beträgt der vorbekannte Schnittwinkel von 40° bis 50°.

In einer Ausführungsform enthält die von der Projektionseinrichtung bereitgestellte Lichtprojektion zumindest zwei Projektionsebenen, die sich unter einem vorbestimmten Winkel schneiden. Die Vorrichtung ist so ausgelegt und angeordnet, dass die Schnittlinie der beiden Projektionsebenen als ein Schnittpunkt in den Bilddaten enthalten ist. Beispielsweise kann der Schnittpunkt der beiden Projektionsebenen in den Bilddaten auf einen Pupillenmittelpunkt gelegt werden. Der Schnittwinkel der beiden Projektionsebenen ist bevorzugt im Wesentlichen 90°. Ist die Projektionsrichtung einstellbar, so wird zum Beispiel eine Projektionsebene im Wesentlichen vertikal und eine im Wesentlichen horizontal im Bezugssystem des Benutzers eingestellt, wobei der Schnittpunkt auf einen Pupillenmittelpunkt angeordnet wird.

Gemäß einer Ausführungsform ist die Benutzerdatenbestimmungseinrichtung so ausgelegt und angeordnet, dass die Benutzerdatenbestimmungseinrichtung die Benutzerdaten aus Bilddaten bestimmt, die mit einer einzigen Aufnahme der Bildaufnahmeeinrichtung erzeugt werden. Die Ermittlung der Daten aus einer einzigen Aufnahme, die von der Lichtprojektion markierte Punkte enthält, ermöglicht eine besonders schnelle Ermittlung der optischen Parameter. In dieser Ausführungsform benötigt die Vorrichtung lediglich eine einzige Bildaufnahmeeinrichtung, die mittels einer einzigen Aufnahme alle Informationen aufnehmen kann, die die Datenverarbeitungseinrichtung zum Ermitteln der optischen Parameter benötigt. Hierbei kann die Benutzerdatenbestimmungseinrichtung Informationen über eine Kalibrierung der Vorrichtung verwenden.

In einer hierzu alternativen Ausführungsform ist die Benutzerdatenbestimmungseinrichtung so ausgelegt und angeordnet, dass sie die Benutzerdaten aus zwei Sätzen von Bilddaten aus zwei unterschiedlichen Aufnahmepositionen bestimmt. Hierbei kann die Vorrichtung entweder zumindest zwei Bildaufnahmeeinrichtungen aufweisen, die im Wesentlichen gleichzeitig Aufnahmen aus zwei Aufnahmepositionen erzeugen, oder eine Bildaufnahmeeinrichtung, die verschwenkbar ist, und zwei Aufnahmen aus unterschiedlichen Aufnahmepositionen aufnimmt. Hierbei enthalten die beiden Sätze von Bilddaten jeweils die Markierung mittels der Lichtprojektion, so dass in den Bilddaten mit Hilfe der Markierung einfach das Korrespondenzproblem gelöst werden kann.

In einer Weiterbildung dieser Ausführungsform ist die Benutzerdatenbestimmungseinrichtung so ausgelegt und angeordnet, dass sie die Markierung durch die Lichtprojektion zur Identifikation korrespondierender Bildpunkte in den beiden Sätzen von Bilddaten nutzt. In dieser Ausführungsform dient die Markierung somit zur vereinfachten Lösung des Korrespondenzproblems bei der 3D-Rekonstruktion. Gemäß einer Ausführungsform stellt die Projektionseinrichtung die Lichtprojektion in einer unsichtbaren Wellenlänge bereit. Um den Benutzer nicht zu irritieren, wird vorzugsweise Strahlung einer Wellenlänge verwendet, die vom menschlichen Auge nicht wahrgenommen wird. Deswegen kann in dieser Ausführungsform die Lichtprojektion ohne Störung des Benutzers so eingestellt werden, dass sie direkt durch die Pupillen des Benutzers verläuft. Die Lichtprojektion kann zum Beispiel im infraroten Wellenlängenbereich liegen, wodurch der Benutzer nicht geblendet wird. Im Spektralbereich oberhalb des sichtbaren Spektrums (wie z.B. im Infraroten) gibt es eine Mehrzahl von Standardkomponenten zur Bildaufnahme und zur Beleuchtung als Massenprodukte. Dadurch kann die entsprechende Vorrichtung kostengünstig realisiert werden. Die unsichtbare Wellenlänge wird von der Bildaufnahmeeinrichtung registriert. Diese ist somit sensitiv in dem von der Lichtprojektion genutzten unsichtbaren Wellenlängenbereich.

Grundsätzlich kann die Lichtprojektion auch in anderen Spektralbereichen realisiert werden, insbesondere im sichtbaren Spektralbereich oder im UV-Bereich.

In einer Ausführungsform kann die Lichtprojektion unterschiedliche Wellenlängen umfassen, die eine weitere Differenzierung und Markierung einzelner Punkte des Systems Kopf mit Brille des Benutzers ermöglichen.

In einer Ausführungsform weist die Vorrichtung eine Vorschauausgabeeinrichtung auf, die anzeigt, auf welchem Teilbereich des Kopfes und oder der Brille des Benutzers die Lichtprojektion ausgerichtet ist. Insbesondere in der Ausführungsform mit der unsichtbaren Wellenlänge wird durch die Vorschauausgabeeinrichtung ein Justieren der Projektionseinrichtung derart ermöglicht, dass sie den gewünschten Teilbereich markiert. Die Vorschauausgabeeinrichtung kann zum Beispiel als Display ausgebildet sein, der die Lichtprojektion in einer auf der Vorschauausgabeeinrichtung sichtbaren Wellenlänge anzeigt. Somit wird die Lichtprojektion lediglich auf der Vorschauausgabeeinrichtung angezeigt, ohne jedoch den Benutzer zu blenden.

Gemäß einer Ausführungsform ist die Vorrichtung als eine tragbare, mobile Vorrichtung ausgebildet. Tragbar und mobil bedeutet hierbei, dass die Vorrichtung maximal 10 kg wiegt, bevorzugt maximal 5 kg, besonders bevorzugt maximal 2 kg. Bei dieser Ausführungsform ist die Vorrichtung so handhabbar, dass sie von einem Bediener getragen werden kann, insbesondere bei Hausbesuchen oder bei Events. Die Vorrichtung kann dabei in der Hand einer Bedienperson getragen und bedient werden. Für den mobilen Einsatz kann zum Beispiel das Objektiv der Bildaufnahmeeinrichtung als Fixierobjekt für den Benutzer verwendet werden, der in Nullblickrichtung auf das Objektiv blickt.

Gemäß einer Ausführungsform weist die Vorrichtung eine Datenausgabeeinrichtung auf, welche zur Ausgabe zumindest eines Teils der bestimmten optischen Parameter des Benutzers ausgelegt ist. Die Datenausgabeeinrichtung kann als Bildschirm und/oder Display ausgelegt sein. Auf der Datenausgabeeinrichtung werden die optischen Parameter angezeigt und können dort ausgelesen werden. Die Datenausgabeeinrichtung kann die optischen Parameter auch als digitale Daten bereitstellen, die von einer anderen Vorrichtung aus ausgelesen werden können.

Ein zweiter Aspekt betrifft ein Verfahren gemäß unabhängigem Anspruch 11.

Das Verfahren eignet sich insbesondere zum Bestimmen der optischen Parameter anhand der Vorrichtung gemäß des ersten Aspekts.

Gemäß einer Ausführungsform werden die Benutzerdaten unter Berücksichtigung einer Kalibrierung der Lichtprojektion relativ zur Positionierung und Ausrichtung einer Bildaufnahmeeinrichtung zur Erzeugung der Bilddaten bestimmt. Hierbei gehen Informationen über die Kalibrierung in die Berechnung der Benutzerdaten ein, zum Beispiel um mit Hilfe epipolargeometrischer Berechnungen die Benutzerdaten im dreidimensionalen Raum zu ermitteln.

In einer Weiterbildung dieser Ausführungsform wird ein Abstand zwischen der Position der Bildaufnahmeeinrichtung und der Position des Benutzers aus den Bilddaten mittels Triangulation unter Berücksichtigung der Kalibrierung abgeschätzt. Dieser Abstand dient als eine wichtige Größe, um die weiteren Benutzerdaten ermitteln zu können.

Gemäß einer Ausführungsform wird die Lichtprojektion bei der Erzeugung der Bilddaten so eingestellt, dass eine Projektionsebene der Lichtprojektion durch die beiden Pupillen des Benutzers verläuft. Die Projektionsebene kann zusätzlich die Fassung der Brille und/oder den Brillenrand schneiden. Diese Einstellung der Lichtprojektion ermöglicht eine besonders einfache Ermittlung der optischen Parameter.

Ein dritter Aspekt betrifft ein Computerprogrammprodukt umfassend Programmteile, welche, wenn geladen in einen Computer, zur Durchführung eines Verfahrens gemäß dem zweiten Aspekt ausgelegt sind.

Die Erfindung wird nachfolgend anhand von Figuren dargestellten Aspekten der Erfindung näher erläutert. Es zeigen:
- Figur 1: schematische Darstellung einer Anordnung von Komponenten einer Vorrichtung zum Bestimmen von optischen Parametern in seitlicher Ansicht;
- Figur 2: perspektivische, schematische Darstellung einer Vorrichtung zum Bestimmen von optischen Parametern,
- Figur 3: perspektivische, schematische Darstellung einer Vorrichtung zum Bestimmen von optischen Parametern mit Benutzer und Brille und
- Figur 4: eine schematische Darstellung einer Aufnahme eines Benutzers mit Brille mit Markierung.

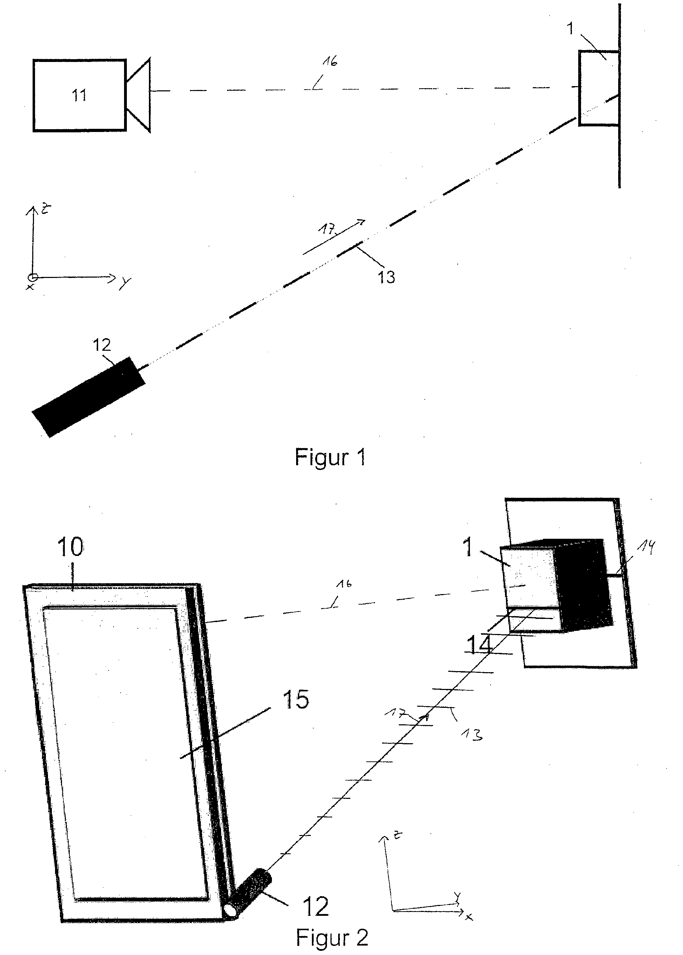

**Figur 1** zeigt eine schematische Darstellung von Komponenten einer Vorrichtung zum Bestimmen von optischen Parametern in seitlicher Ansicht. Als Komponenten der Vorrichtung sind in Figur 1 eine Bildaufnahmeeinrichtung 11 und eine Projektionseinrichtung 12 gezeigt. Die Bildaufnahmeeinrichtung 11 kann zum Beispiel als eine Kamera ausgebildet sein, insbesondere als eine Digitalkamera. Die Projektionseinrichtung 12 kann als Lichtquelle ausgebildet sein, insbesondere als eine Linienlichtquelle.

Sowohl die Bildaufnahmeeinrichtung 11 als auch die Projektionseinrichtung 12 sind auf ein Objekt 1 ausgerichtet, das lediglich zur Veranschaulichung des Messverfahrens dient und im Wesentlichen quaderförmig ausgebildet ist.

Die Bildaufnahmeeinrichtung 11 und die Projektionseinrichtung 12 sind zueinander kalibriert. Dies bedeutet, dass sie u. A. einen vorbekannten Abstand voneinander aufweisen. In dem in Figur 1 eingezeichneten Koordinatensystem weisen die Bildaufnahmeeinrichtung 11 und die Lichtprojektionseinrichtung 12 einen vorbekannten Abstand in Z-Richtung auf. Zur Kalibrierung gehört weiterhin Informationen über die Ausrichtung der Bildaufnahmeeinrichtung 11 und der Lichtprojektionseinrichtung 12. In dem Koordinatensystem der Figur 1 ist die Bildaufnahmeeinrichtung 11 so ausgerichtet, dass der Richtungsvektor der der optischen Achse 16 der Bildaufnahmeeinrichtung 11 im Wesentlichen mit der Y-Richtung zusammenfällt. Die Projektionseinrichtung 12 ist in eine Projektionsrichtung 17 ausgerichtet, die sowohl eine Y- als auch eine Z-Komponente, jedoch keine X-Komponente aufweist.

Der Richtungsvektor der optischen Achse 16 und der Richtungsvektor der Projektionsrichtung 17 schneiden sich unter einem vorbekannten Winkel. Die Größe des Winkels und der Abstand der Bildaufnahmeeinrichtung 11 von der Projektionseinrichtung 12 sind Bestandteil der Kalibrierung der Vorrichtung. Der Abstand der Bildaufnahmeeinrichtung 11 von der Lichtprojektionseinrichtung 12 dient als Triangulationsbasis, mit der der Abstand des Objektes 1 von der Bildaufnahmeeinrichtung 11 ermittelt werden kann, im gezeigten Ausführungsbeispiel ein Abstand in Y-Richtung.

Die Projektionseinrichtung 12 erzeugt eine Lichtprojektion, die in Projektionsrichtung 17 abgestrahlt wird und einen Teilbereich des Objekts 1 markiert. Die Komponenten der Vorrichtung sind so angeordnet, dass diese Markierung in den von der Bildaufnahmeeinrichtung 11 aufgenommenen Bilddaten enthalten ist. Mit anderen Worten ist die von der Lichtprojektion hervorgerufene Markierung in der Aufnahme sichtbar.

In den Figuren ist die Z-Richtung im Wesentlichen vertikal im Bezugssystem des Benutzers angeordnet, die Y-Richtung im Wesentlichen horizontal von der Bildaufnahmeeinrichtung 11 hin zum Benutzer 2 bzw. dem Objekt 1, und die X-Richtung im Wesentlichen horizontal durch die beiden Pupillenmittelpunkte des Benutzers 2 und/oder senkrecht zur optischen Achse 16 der Bildaufnahmeeinrichtung 11.

**Figur 2** zeigt in einer perspektivischen, schematischen Darstellung eine Vorrichtung 10 mit den beiden bereits in Figur 1 gezeigten Komponenten. Dabei ist die Bildaufnahmeeinrichtung 11 von der Rückseite der Vorrichtung 10 verdeckt, so dass lediglich deren optische Achse 16 in Figur 2 gezeigt ist. Die Projektionseinrichtung 12 projiziert eine Markierung 14 in Form einer Linie auf das Objekt 1. Die Markierungslinie erstreckt sich in X-Richtung und ist deswegen in Figur 1 nicht sichtbar. Die Projektionseinrichtung 12 emittiert Licht in einer Projektionsebene 13 entlang der Projektionsrichtung 17, die auf dem Objekt 1 als Linie erscheint. Dabei weist der Richtungsvektor der Projektionsrichtung 17 von der Projektionseinrichtung 12 zum Objekt 1. Der emittierte Lichtstrahl der Lichtprojektion weist eine gewisse Breite auf (z.B. im Objektabstand von der Bildaufnahmeeinrichtung zumindest 10 cm), jedoch im Wesentlichen keine Höhe. Dadurch ergibt sich die Form der Lichtprojektion zu der Projektionsebene 13. Dabei liegt der Richtungsvektor der Projektionsrichtung 17 in der Projektionsebene 13. Weiterhin weist die Projektionsebene 13 eine im Wesentlichen horizontale Komponente auf, die senkrecht zu der in Figur 1 gezeigten Zeichenebene angeordnet ist. Deswegen ist die Projektionsebene 13 in Figur 1 von der Seite als Linie in Projektionsrichtung 17 gezeigt.

Die von der Projektionseinrichtung 12 erzeugte Markierung 14 weist im Wesentlichen die Form einer durchbrochenen Linie auf. In der von der Bildaufnahmeeinrichtung 11 aufgenommenen Aufnahme erscheint ein Teil der Markierung 14 (in Z-Richtung gesehen) weiter unten, ein anderer Teil weiter oben (vgl. Figur 2). Näher an der Bildaufnahmeeinrichtung 11 angeordnete Teile des Objektes 1 werden weiter unten (in negativer Z-Richtung) von der Lichtprojektion in Form einer Linie geschnitten und sind daher in dem mit der Bildaufnahmeeinrichtung 11 aufgenommenen Bild ebenfalls weiter unten angeordnet. Wie in Figur 2 zu sehen, ist der Teil der Markierung 14, der auf die hinter dem Objekt 1 angeordnete Wand projiziert wird, in Z-Richtung weiter oben angeordnet als derjenige Teil der Markierung 14, der auf dem in negative Y-Richtung herausragenden Teil des Objektes 1 projiziert ist.

Somit ist der Abstand der Punkte des markierten Teilbereichs von der Bi Idaufnahmeeinrichtung 11 und/oder der Vorrichtung 10 in den Bilddaten dadurch bestimmbar, dass die Position der markierten Punkte in der Aufnahme bestimmt wird, in der gezeigten Ausführungsform insbesondere die Position auf der Z-Achse. Über die Kalibration der Vorrichtung 10 ist der Abstand (in Y-Richtung) der markierten Punkte auf dem Objekt 1 in den Bilddaten von der Bildaufnahmeeinrichtung 11 zum Objekt 1 somit mittels Triangulation berechenbar.

Wie in Figur 2 gezeigt, weist die Vorrichtung 10 eine Anzeige 15 auf, die auf der dem Objekt 1 abgewandten Seite der Vorrichtung 10 angeordnet ist. Die Anzeige 15 kann zum Beispiel als Bildschirm oder Display ausgebildet sein. Die Anzeige 15 kann zum Beispiel als Datenausgabeeinrichtung verwendet werden, die die von der Vorrichtung 10 bestimmten optischen Parameter anzeigt.

Die Anzeige 15 kann zusätzlich oder alternativ dazu ausgelegt und vorgesehen sein, eine Vorschau der Bilddaten mitsamt der von der Projektionseinrichtung 12 erzeugten Markierung 14 anzuzeigen. Die Anzeige 15 erzeugt somit eine Vorschau des aufzunehmenden Bildes und dient als Vorschauausgabeeinrichtung. Dies ist besonders günstig, wenn die Projektionseinrichtung 12 Strahlung in einer nicht sichtbaren Wellenlänge wie zum Beispiel im Infraroten erzeugt, die für einen menschlichen Benutzer nicht sichtbar ist. Zum Einstellen und/oder Ausrichten der Vorrichtung 10 relativ zum Objekt 1 kann eine Bedienperson wie zum Beispiel ein Optiker auf der Anzeige 15 sehen, welchen Teilbereich des Objekts 1 die Projektionseinrichtung 12 markiert.

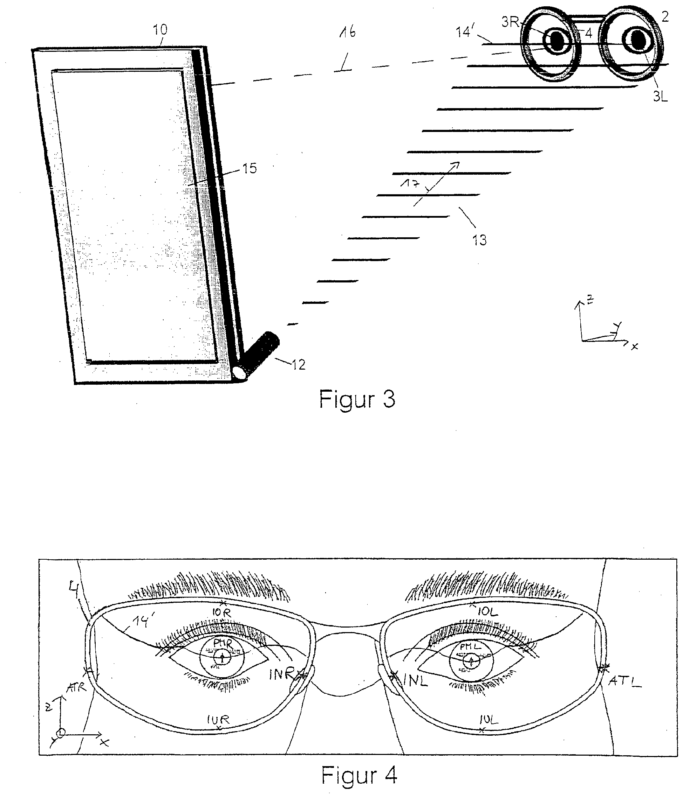

**Figur 3** zeigt die aus Figur 2 bekannte Vorrichtung 10 in einer perspektivischen, schematischen Darstellung aus einer ähnlichen Perspektive wie in Figur 2. Im Unterschied zu dem in Figur 2 schematisch dargestellten Objekt 1 ist in Figur 3 schematisch ein Benutzer 2 mit einer Brille 4 angeordnet. Bei der Aufnahme der Bilddaten ist die Projektionsrichtung 17 der Projektionseinrichtung 12 und insbesondere die Projektionsebene 13 so ausgerichtet, dass die dadurch erzeugte Markierung 14' durch die beiden Pupillenmitten des Benutzers 2 verläuft. Die Markierung 14' verläuft durch die Mitte der rechten Pupille 3R des Benutzers 2 und die Mitte der linken Pupille 3L des Benutzers 2. Weiterhin schneidet die Markierung 14' die Fassung der Brille 4 an mehreren nasalen und temporalen Positionen der Fassung, insbesondere an inneren und äußeren nasalen und temporalen Fassungspunkten der Brille 4. Ortsinformationen zu einige dieser Punkte können als Benutzerpunkte bevorzugt bestimmt werden, um die optischen Parameter besonders günstig zu berechnen.

Dabei zeigt Figur 3 die Markierung 14' lediglich schematisch. In den von der Bildaufnahmeeinrichtung 11 erzeugten Bilddaten erscheint die Markierung 14' ähnlich wie die in Figur 2 schematisch dargestellte Markierung 14 als eine Linie, die insbesondere beim Übergang von Fassung zum Kopf des Benutzers unterbrochen ist, da die Fassung der Brille 4 näher an der Bildaufnahmeeinrichtung 11 angeordnet ist als der markierte Teilbereich des Kopfes wie z.B. die beiden Pupillen 3L und 3R des Benutzers 2.

Aus den unterschiedlichen Z-Positionen der Markierung 14' in der von der Bildaufnahmeeinrichtung 11 aufgenommenen Aufnahme kann mittels Triangulation der Abstand der markierten Punkte auf der Fassung der Brille 4 sowie der markierten Punkte auf dem Kopf des Benutzers 2 zur Bildaufnahmeeinrichtung 11 berechnet werden. Dadurch können Benutzerdaten im dreidimensionalen Raum errechnet werden. Benutzerdaten können mittels einer Benutzerdatenbestimmungseinrichtung errechnet werden, die auf einen Mikroprozessor der Vorrichtung 10 zurückgreifen kann. Aus den Benutzerdaten können von einer Parameterbestimmungseinrichtung optische Parameter des Benutzers 2 ermittelt werden.

Die Vorrichtung 10 nutzt eine aktive Beleuchtung, um in einem Aufbau zur Videozentrierung mittels einer 3D-Rekonstruktion die optischen Parameter wie Zentrierdaten und individuelle Parameter des Benutzers zu ermitteln.

Alternativ zu der in den Figuren gezeigten Vorrichtung kann auch ein Stereokamerasystem verwendet werden, das Bilddaten aus zwei unterschiedlichen Perspektiven erzeugt, wie zum Beispiel aus dem Dokument DE 10 2005 003 699 A1 bekannt. Hierbei kann die durch eine zusätzliche Projektionseinrichtung 12 bereitgestellte aktive Beleuchtung zum Lösen und/oder beschleunigten Lösen des Korrespondenzproblems verwendet werden, das sich ergibt, wenn in den Aufnahmen aus zwei unterschiedlichen Perspektiven zueinander korrespondierende Punkte identifiziert werden müssen.

Der Benutzer 2 kann als Fixierobjekt die Bildaufnahmeeinrichtung 11 oder einen anderen Punkt der Vorrichtung 10 verwenden, den er bei der Aufnahme der Bilddaten fixiert. Der Abstand des verwendeten Fixierobjekts zu den Pupillen des Benutzers kann nachträglich zur Konvergenzkorrektur verwendet werden. Alternativ kann ein bezüglich des Benutzers 2 bestimmbarer Punkt als Fixierpunkt verwendet werden, wie z. B. die Nasenwurzel des Benutzers in einem Spiegelbild, wobei das Spiegelbild durch einen Spiegel bereitgestellt wird, der zur Bildaufnahmeeinrichtung 11 in einer vorbekannten Position gefestigt ist.

### Ausführungsform mit einem Stereokamerasystem

In einem wie oben erwähnten Stereokamerasystem als Vorrichtung zum Bestimmen von optischen Parametern des Benutzers kann die Markierung dazu verwendet werden, in gleichzeitig oder zeitversetzt zueinander aufgenommenen Bilddaten zueinander korrespondierende Punkte zu lokalisieren. Dies kann durch maschinelle und/oder manuelle Bildverarbeitung erfolgen. Dabei kann die manuelle Auswertung auf den ersten Satz Bilddaten reduziert werden, wenn Epipolarlinien der als Linie projizierten Markierung in den beiden Sätzen von Bilddaten nicht übereinstimmen. Aus einem in dem ersten Satz Bilddaten ausgewählten Punkt ist der korrespondierende Punkt in dem zweiten Satz Bilddaten durch die Epipolarlinie und dem Schnittpunkt mit der projizierten Markierung festgelegt. Dieser Schnittpunkt kann zum Beispiel durch automatische Bildverarbeitung ermittelt werden.

Das Stereokamerasystem kann auch lediglich eine Bildaufnahmeeinrichtung aufweisen, die zeitversetzt zwei Sätze von Bilddaten aus unterschiedlichen Blickwinkeln auf den Benutzer aufnimmt. In diesem Fall ist die Kalibrierung der Kamerapositionen nicht unbedingt vorab bekannt und kann aus den aufgenommenen Bilddaten in einem Verfahrensschritt ermittelt werden. Zur Kalibrierung kann zum Beispiel die Position der Pupillen 3L und/oder 3R relativ zur Fassung der Brille verwendet werden. Da sich die Pupille und die Fassung in unterschiedlichen Abständen zu den beiden Positionen der Bildaufnahmeeinrichtung befinden, ergibt sich ein entsprechender Versatz je nach Beobachtungsrichtung.

Für die Bestimmung der Orientierung können zum Beispiel neben den beiden Pupillen des Benutzers fünf charakteristische Punkte auf der Fassung verwendet werden, die in den beiden Sätzen von Bilddaten jeweils selektiert werden. Ist als Fixierobjekt ein sich bewegender Punkt vorgegeben, so können anstatt der beiden Pupillen des Benutzers zwei andere nicht veränderliche Punkte am Benutzer und/oder der Fassung ausgewählt werden. Aus diesen insgesamt sieben Punkten kann über den aus der Epipolargeometrie bekannten 7-Punkte Algorithmus eine 3D-Rekonstruktion der Punkte aus den aufgenommenen Bilddaten erfolgen.

### Ausführungsform mit einem Satz Bilddaten

In der Ausführungsform der Vorrichtung, wie sie in den Figuren dargestellt ist, werden die optischen Parameter aus einer einzigen Aufnahme (also aus einem einzigen Satz Bilddaten) ermittelt. Hierbei sind die Bildaufnahmeeinrichtung 11 und die Projektionseinrichtung 12 zueinander und vorrichtungsintern kalibriert. Aus der von der Projektionseinrichtung 12 erzeugten Markierung 14 bzw. 14' in den Bilddaten wird eine Berechnung der Benutzerdaten im Dreidimensionalen ermöglicht. Dabei kann eine Ausrichtung der Markierung 14, 14' eingestellt werden, in der die als Linie erzeugte Markierung im Wesentlichen senkrecht zur Epipolarebene angeordnet ist. Dadurch kann als Triangulationsbasisobjekt ein Dreieck verwendet werden, wobei die Triangulationsbasis im Wesentlichen vertikal (in den Figuren in Z-Richtung) ausgerichtet ist.

Durch eine maschinelle und/oder automatische Bildverarbeitung der Bilddaten und/oder eine manuelle Selektion der beiden Pupillenmittelpunkte kann aus den Bilddaten zum Beispiel der Pupillenabstand im Dreidimensionalen ermittelt werden. Aus den markierten Punkten und den erzeugten Benutzerdaten können weitere Parameter ermittelt werden, wie zum Beispiel der Fassungsscheibenwinkel und der horizontale Schnitt der Topographie des Auges, sowie näherungsweise der Hornhautscheitelabstand, die Scheibenlänge links, die Scheibenlänge rechts etc. Hierbei erfolgt eine Auswertung der Bilddaten einer einzigen Aufnahme entlang der projizierten Markierung (in der gezeigten Ausführungsform entlang der Linie). Dadurch ist die Auswertung sehr schnell durchführbar.

Durch eine zusätzliche, zweite Projektion einer weiteren, zweiten Linie, zum Beispiel einer vertikalen Linie (in den Figuren nicht dargestellt), können weitere optische Parameter bestimmt werden. Im Gegensatz zur im Wesentlichen horizontal ausgerichteten ersten Markierung 14 und 14' ist diese z.B. von einer zweiten Projektionseinrichtung erzeugte, im Wesentlichen vertikal ausgerichtete zweite Markierung mittels einer zweiten Triangulationsbasis kalibrierbar. Dazu kann z.B. ein horizontaler Abstand (in der gezeigten Ausführungsform in X-Richtung) der zweiten Projektionseinrichtung zu der Bildaufnahmeeinrichtung 11 vorbekannt sein. Jedenfalls können ein Richtungsvektor der zweiten Projektionsrichtung relativ zur optischen Achse 16 sowie eine Position der zweiten Projektionseinrichtung relativ zur Bildaufnahmeeinrichtung 11 als weitere Information der Kalibrierung vorbekannt sein.

Durch diese weitere, im Wesentlichen vertikale Markierung können weitere optische Parameter berechnet werden, wie zum Beispiel die Vorneigung, die Scheibenhöhe, der Hornhautscheitelabstand, usw.

Dabei kann diese zweite Markierung so positioniert werden, dass sie über einer Pupille angeordnet ist, also im Wesentlichen vertikal durch die rechte oder linke Pupille des Benutzers verläuft.

In einer Ausführungsform können mehrere, insbesondere zwei solcher vertikalen Markierungen in Form einer Linie auf das System Kopf mit Brille projiziert werden. In einem Ausführungsbeispiel werden zwei zueinander parallele vertikale Markierungen in Form einer Linie verwendet, deren Abstand zueinander in einem vorbestimmten Messabstand von der Kamera im Wesentlichen der typischen Pupillendistanz entspricht, das heißt ca. 64 mm beträgt. Dadurch können zusätzliche optische Parameter bestimmt werden, und zwar für jedes Auge getrennt voneinander.

Ein Auslösen der Bildaufnahmeeinrichtung, also ein Aufnehmen der Bilddaten, kann automatisiert erfolgen. Hierbei kann eine Detektion, also ein Aufnehmen der Bilddaten, während der Positionierung der Vorrichtung ausgeführt werden, wenn geeignete Auslösebedingungen, wie zum Beispiel automatisch erkannte Pupillenpositionen, erfüllt und detektiert werden.

Bei der in den Figuren dargestellten Vorrichtung 10 weist die von der Projektionseinrichtung 12 erzeugte Lichtprojektion eine Projektionsrichtung 17 auf, die auf einem Benutzer 2 eine im Wesentlichen horizontale Markierung 14' erzeugt. Die von der Projektionseinrichtung 12 erzeugte Markierung ist hierbei bevorzugt parallel zu den Zeilen in den Bilddaten der Bildaufnahmeeinrichtung ausgerichtet, insbesondere wenn diese als Digitalkamera ausgebildet ist. Dies ermöglicht eine einfache Triangulationsbasis und Abstandsbestimmung der markierten Bildpunkte in den Bilddaten.

Die Markierung ermöglicht ein vereinfachtes Auswählen einzelner Punkte in den Bilddaten, insbesondere an Schnittpunkten der Markierung mit dem Fassungsrand und/oder mit den Pupillenmittelpunkten.

**Figur 4** zeigt eine schematische Darstellung einer Aufnahme vom System Kopf eines Benutzers mit Brille 4, die von der Vorrichtung 10 aufgenommen wurde, die in den Figuren 1-3 gezeigt ist. Die von der Bildaufnahmeeinrichtung 11 aufgenommenen Bilddaten können der in Figur 4 schematisch gezeigten Aufnahme entsprechen.

Die Aufnahme enthält Bilddaten beider Pupillen des Benutzers sowie der Fassung der Brille 4. Die Aufnahme wurde von der Vorrichtung 10 entgegen der Nullblickrichtung des Benutzers aufgenommen und ist als zweidimensionale Aufnahme ausgebildet. Die Bilddaten enthalten die von der Lichtprojektionseinheit 12 mittels der Projektionsebene 13 erzeugte Markierung 14'. Dabei weist die Markierung 14' in der Aufnahme im Wesentlichen die Form einer Linie auf. Um die unterschiedliche Höhe der Markierung 14' (also die unterschiedliche Position auf der Z-Achse) in den Bilddaten zu verdeutlichen, ist die Variation der projizierten Linie in Z-Achsenrichtung in Figur 4 übertrieben dargestellt. In einer realen Aufnahme würde die Markierung 14' eher im Wesentlichen die Form einer Linie aufweisen.

Beim Auslösen der Aufnahme war die projizierte Markierung 14' so ausgerichtet, dass sie durch die beiden Pupillenmittelpunkte PMR und PML des Benutzers verläuft.

Der Verlauf der Markierung 14' ist im Folgenden beschrieben von links nach rechts durch die in Figur 4 dargestellte Aufnahme, also im Wesentlichen in positiver X-Richtung. Die Markierung 14' verläuft dort vom rechten äußeren temporalen Fassungspunkt ATR über den Fassungsrand zum rechten inneren temporalen Fassungspunkt (nicht eigens markiert). Von dieser Stelle "springt" die Markierung 14' in der Aufnahme in positive Z-Richtung, also nach oben, auf eine Position rechts (in negativer X-Richtung) des rechten Auges des Benutzers. Von dort verläuft die Markierung 14' über den rechten Pupillenmittelpunkt PMR in Richtung Nasenwurzel. Von dort "springt" die Markierung 14' auf die Fassung an einer nasalen Position, genauer auf den rechten inneren nasalen Fassungspunkt INR, zum rechten äußeren nasalen Fassungspunkt (nicht markiert) und nach einem weiteren "Sprung" weiter über die Nase des Benutzers.

Von dort verläuft die Markierung 14' im Wesentlichen spiegelsymmetrisch über die linke Augen- und Fassungshälfte, insbesondere den linken inneren nasalen Fassungspunkt INL, den linken Pupillenmittelpunkt PML bis zum linken äußeren temporalen Fassungspunkt ATL.

Die Bilddaten der in Figur 4 gezeigten Aufnahme enthalten z.B. Pixelpositionen jedes Bildpunktes. Aus diesen X- und Z-Pixelpositionen können mittels Triangulation unter Verwendung der Kalibrierung die X-, Y- und Z-Koordinaten jedes markierten Punktes ermittelt werden. So kann z.B. die Y-Koordinate mittels Triangulation und Informationen über die Kalibrierung für die markierten Punkte in Abhängigkeit von der jeweiligen Z-Pixelposition des markierten Punkts ermittelt werden. Auch die Umrechnung der realen X- und Z-Koordinate des markierten Punkts aus den X- und Z-Pixelpositionen in den Bilddaten kann abhängig von der Y-Koordinate mittels eines Skalierungsfaktors berechnet werden. Somit können für jeden markierten Punkt, insbesondere für die sechs gezielt markierten Punkte ATR, PMR, INR, INL, PML und ATL Ortsinformationen im dreidimensionalen Raum bestimmt werden.

Alle diese sechs Punkte ATR, PMR, INR, INL, PML und ATL sind in den Bilddaten mittels der von der Projektionseinheit 12 bereitgestellten Markierungslinie gezielt markiert. Wie oben erläutert können aus den dreidimensionalen Ortsinformationen dieser sechs ausgewählten Punkte des Systems Kopf mit Brille die optischen Parameter Pupillenabstand, Scheibenlänge links, Scheibenlänge rechts, Fassungsscheibenwinkel, der horizontale Schnitt der Topographie des Auges, näherungsweise der Hornhautscheitelabstand, etc. berechnet werden.

Mit einer zusätzlichen zweiten Lichtprojektion und somit zweiten Markierung in vertikaler Richtung können zusätzlich die Punkte rechter innerer Fassungspunkt oberhalb der Pupille IOR, nochmal rechter Pupillenmittelpunkt PMR und rechter innerer Fassungspunkt unterhalb der Pupille IUR markiert werden. In Figur 4 sind diese Punkte gezeigt, allerdings ohne die entsprechende zweite Markierung.

Unter Berücksichtigung von Informationen über die Kalibrierung dieser zweiten Lichtprojektion (z.B. in horizontaler Richtung) können für jeden von der zweiten Lichtprojektion markierten Punkt, insbesondere für die drei gezielt markierten Punkte IOR, PMR und IUR Ortsinformationen im dreidimensionalen Raum bestimmt werden. Mit den dreidimensionalen Ortsinformationen dieser zusätzlichen Punkte können die zusätzlichen optischen Parameter Vorneigung, Scheibenhöhe, Hornhautscheitelabstand, usw. für das rechte Auge berechnet werden.

Analog dazu können mit einer zusätzlichen dritten Lichtprojektion parallel zur zweiten Lichtprojektion in vertikaler Richtung die zusätzlich die Punkte linker innerer Fassungspunkt oberhalb der Pupille IOL, nochmal linker Pupillenmittelpunkt PML und linker innerer Fassungspunkt unterhalb der Pupille IUL markiert werden. In Figur 4 sind diese Punkte gezeigt, ohne die entsprechende dritte Markierung.

Unter Berücksichtigung von Informationen über die Kalibrierung dieser dritten Lichtprojektion (z.B. in horizontaler Richtung) können für jeden von der dritten Lichtprojektion markierten Punkt, insbesondere für die drei gezielt markierten Punkte IOL, PML und IUL Ortsinformationen im dreidimensionalen Raum bestimmt werden. Mit den dreidimensionalen Ortsinformationen dieser zusätzlichen Punkte können die zusätzlichen optischen Parameter Vorneigung, Scheibenhöhe, Hornhautscheitelabstand, usw. für das linke Auge berechnet werden.

Beim Auslösen der Aufnahme ist diese optionale zweite und dritte Markierungslinie so ausgerichtet, dass jeweils eine Markierungslinie vertikal (parallel zur Z-Achse) durch jeweils einen Pupillenmittelpunkt PMR bzw. PML des Benutzers verläuft.

Nachfolgend ist die Berechnung zweier beispielhafter optischer Parameter aus den dreidimensionalen Ortsinformationen der oben benannten Punkte beschrieben:
Der optische Parameter "Pupillenabstand" kann als Länge zwischen den Punkten PMR und PML im dreidimensionalen Raum berechnet werden.. Zusätzlich kann eine Aufteilung des Pupillenabstands in rechten Pupillenabstand und linken Pupillenabstand als zusätzliche optische Parameter erfolgen. Dazu kann eine Pupillenmittenebene definiert werden, die den gleichen Abstand zu den Punkten INL und INR hat und somit zwischen diesen beiden Punkten angeordnet ist. Der Schnittpunkt dieser Pupillenmittenebene mit einer Verbindungslinie der beiden Punkte PMR und PML stellt eine Aufteilung des optischen Parameters "Pupillenabstands" in den rechten Pupillenabstand (als Strecke dieses Schnittpunkts zu PMR) und den linken Pupillenabstand (als Strecke dieses Schnittpunkts zu PML) bereit.

Der optische Parameter "Fassungsscheibenwinkel" kann in einer horizontalen Projektion aus dem Winkel zwischen den Geraden berechnet werden, die durch die Strecken ATR-INR und ATL-INL gegeben sind.

Allgemein können für die Berechnung der optischen Parameter auch mehr und/oder andere als die zehn oben explizit genannten Punkte verwendet werden. Für die optischen Parameter "Scheibenlänge" und "Scheibenhöhe" ist mit den genannten zehn Punkten nur eine näherungsweise Berechnung möglich. Für eine genaue Berechnung dieser Parameter kann das eingangs aufgeführte Kastenmaß verwendet werden, das in den Benutzerdaten berücksichtigt werden kann.

Zur Ermittlung des Kastenmaßes als Bestandteil der Benutzerdaten kann (z.B. von der Benutzerdatenbestimmungseinrichtung) eine Selektion einer Begrenzung des Brillenglases durch ein Viereck in den Bilddaten vorgenommen werden. Dabei können sich Begrenzungslinien der Begrenzung nur anhand vordefinierter Richtungen verschieben lassen. So kann eine obere Begrenzungslinie als Horizontale in der Scheibenebene angeordnet sein und entsprechend als Projektion in den Bilddaten dargestellt werden. Diese obere Begrenzungslinie kann z.B. nur entlang einer vertikalen Richtung verschoben werden. Für eine innere Begrenzungslinie kann analog nur eine horizontale Verschiebung vorgesehen sein, wobei die innere Begrenzungslinie als vertikale Linie in der Scheibenebene dargestellt wird. Eine Bestimmung der dreidimensionalen Ortsinformation der Eckpunkte des Kastenmaßes kann über die bereits selektierten Punkte erfolgen, zu denen dreidimensionale Ortsinformationen vorliegen, sowie durch diese Punkte verknüpfende Skalierungsfaktoren.

### Bezugszeichenliste

- 1: Objekt
- 2: Benutzer
- 3R: rechte Pupille
- 3L: linke Pupille
- 4: Brille
- 10: Vorrichtung
- 11: Bildaufnahmeeinrichtung
- 12: Projektionseinrichtung
- 13: Projektionsebene
- 14: Markierung
- 14': Markierung
- 15: Anzeige
- 16: optische Achse
- 17: Projektionsrichtung
- PMR: rechter Pupillenmittelpunkt
- PML: linker Pupillenmittelpunkt
- ATR: rechter äußerer temporaler Fassungspunkt
- ATL: linker äußerer temporaler Fassungspunkt
- INR: rechter innerer nasaler Fassungspunkt
- INL: linker innerer nasaler Fassungspunkt
- IOR: rechter innerer Fassungspunkt oberhalb der Pupille
- IOL: linker innerer Fassungspunkt oberhalb der Pupille
- IUR: rechter innerer Fassungspunkt unterhalb der Pupille
- IUL: linker innerer Fassungspunkt unterhalb der Pupille

## Patentansprüche

1. Vorrichtung (10) zum Bestimmen von optischen Parametern eines Benutzers (2) mit einer am Kopf des Benutzers (2) in Gebrauchsstellung angeordneten Brille (4), mit
- zumindest einer Projektionseinrichtung (12), welche ausgelegt und angeordnet ist, einen Teilbereich des Kopfes des Benutzers (2) und/oder der Brille (4) des Benutzers mit einer Lichtprojektion zu markieren,
- zumindest einer Bildaufnahmeeinrichtung (11), welche ausgelegt und angeordnet ist, Bilddaten zumindest von dem markierten Teilbereich des Kopfes des Benutzers (2) und/oder der Brille (4) des Benutzers zu erzeugen, und
- einer Datenverarbeitungseinrichtung mit
- einer Benutzerdatenbestimmungseinrichtung, welche ausgelegt ist, anhand der erzeugten Bilddaten Benutzerdaten von dem markierten Teilbereich des Kopfes und/oder der Brille (4) zu bestimmen, wobei die Benutzerdaten Ortsinformationen im dreidimensionalen Raum von Punkten des Teilbereichs des Kopfes und/oder der Brille (4) umfassen und
- einer Parameterbestimmungseinrichtung, welche ausgelegt ist, anhand der Benutzerdaten optische Parameter des Benutzers (2) zu bestimmen;
wobei die Projektionseinrichtung (12) so ausgelegt und angeordnet ist, dass durch die Lichtprojektion in den Bilddaten gezielt einzelne Punkte auf dem Kopf und/oder der Brille (4) des Benutzers derart markiert werden,
dass in den Bilddaten zumindest einer der folgenden Benutzerpunkte von der Lichtprojektion in Form einer Linie, einer Linienkreuzung und/oder eines Punktes gezielt markiert ist:
- ein Pupillenmittelpunkt (PMR, PML),
- ein äußerer temporaler Fassungspunkt (ATR, ATL),
- ein innerer nasaler Fassungspunkt (INR, INL),
- ein innerer Fassungspunkt oberhalb der Pupille (IOR, IOL) und/oder
- ein innerer Fassungspunkt unterhalb der Pupille (IUR, IUL).

2. Vorrichtung nach Anspruch 1, wobei die Projektionseinrichtung (12) relativ zur Bildaufnahmeeinrichtung (11) kalibriert ist und die Benutzerdatenbestimmungseinrichtung Informationen über diese Kalibrierung zur Bestimmung der Benutzerdaten benutzt.

3. Vorrichtung nach Anspruch 1 oder 2, wobei die Projektionseinrichtung (12) eine einstellbare Projektionsrichtung (17) aufweist.

4. Vorrichtung nach einem der vorangehenden Ansprüche, wobei die von der Projektionseinrichtung (12) bereitgestellte Lichtprojektion zumindest eine Projektionsebene (13) enthält, die auf dem beleuchteten Teilbereich des Kopfes und/oder der Brille (4) des Benutzers zumindest teilweise eine Linie (14) projiziert.

5. Vorrichtung nach Anspruch 4, wobei die Projektionseinrichtung (12) relativ zur Bildaufnahmeeinrichtung (11) so kalibriert ist, dass sich die optische Achse (16) der Bildaufnahmeeinrichtung (11) und die Projektionsebene (13) unter einem vorbekanntem Schnittwinkel von zumindest 10° beträgt und maximal 70° schneiden.

6. Vorrichtung nach einem der vorangegangenen Ansprüche, wobei die von der Projektionseinrichtung (12) bereitgestellte Lichtprojektion zumindest zwei Projektionsebenen enthält, die sich unter einem vorbestimmten Winkel schneiden.

7. Vorrichtung nach einem der vorangegangenen Ansprüche, wobei die Benutzerdatenbestimmungseinrichtung so ausgelegt und angeordnet ist, dass sie die Benutzerdaten aus Bilddaten bestimmt, die mit einer einzigen Aufnahme der Bildaufnahmeeinrichtung (11) erzeugt werden;
oder
wobei die Benutzerdatenbestimmungseinrichtung so ausgelegt und angeordnet ist, dass sie die Benutzerdaten aus zwei Sätzen von Bilddaten aus unterschiedlichen Aufnahmepositionen bestimmt und die Markierung (14; 14') durch die Lichtprojektion zur Identifikation korrespondierender Bildpunkte in den beiden Sätzen von Bilddaten nutzt.

8. Vorrichtung nach einem der vorangegangenen Ansprüche, wobei die Projektionseinrichtung (12) die Lichtprojektion in einer unsichtbaren Wellenlänge bereitstellt.

9. Vorrichtung nach Anspruch 8, mit einer Vorschauausgabeeinrichtung (15), die anzeigt, auf welchen Teilbereich des Kopfes und/oder der Brille (4) des Benutzers die unsichtbare Lichtprojektion ausgerichtet ist.

10. Vorrichtung nach einem der vorangegangenen Ansprüche, wobei die Vorrichtung (10) als eine tragbare, mobile Vorrichtung ausgebildet ist.

11. Verfahren zum Bestimmen von optischen Parametern eines Benutzers (2) mit einer am Kopf des Benutzers (2) in Gebrauchsstellung angeordneten Brille (4), wobei:
- ein Teilbereich des Kopfes des Benutzers (2) und/oder der Brille (4) des Benutzers (2) mit einer Lichtprojektion markiert wird,
- Bilddaten von zumindest dem markierten Teilbereich des Kopfes des Benutzers (2) und/oder der Brille (4) des Benutzers erzeugt werden,
- anhand der erzeugten Bilddaten Benutzerdaten von dem markierten Teilbereich des Kopfes und/oder der Brille (4) bestimmt werden, wobei die Benutzerdaten Ortsinformationen im dreidimensionalen Raum von Punkten des Teilbereichs des Kopfes und/oder der Brille (4) umfassen,
- anhand der Benutzerdaten optische Parameter des Benutzers (2) bestimmt werden,
- durch die Lichtprojektion in den Bilddaten gezielt einzelne Punkte auf dem Kopf und/oder der Brille (4) des Benutzers derart markiert werden, dassin den Bilddaten zumindest einer der folgenden Benutzerpunkte von der Lichtprojektion in Form einer Linie, einer Linienkreuzung und/oder eines Punktes gezielt markiert wird:
- ein Pupillenmittelpunkt (PMR, PML),
- ein äußerer temporaler Fassungspunkt (ATR, ATL),
- ein innerer nasaler Fassungspunkt (INR, INL),
- ein innerer Fassungspunkt oberhalb der Pupille (IOR, IOL) und/oder
- ein innerer Fassungspunkt unterhalb der Pupille (IUR, IUL).

12. Verfahren nach Anspruch 11, wobei die Benutzerdaten unter Berücksichtigung einer Kalibrierung der Lichtprojektion relativ zur Positionierung und Ausrichtung einer Bildaufnahmeeinrichtung (11) zur Erzeugung der Bilddaten bestimmt werden.

13. Verfahren nach Anspruch 12, wobei ein Abstand zwischen der Position der Bildaufnahmeeinrichtung (11) und der Position des Benutzers (2) aus den Bilddaten mittels Triangulation unter Berücksichtigung der Kalibrierung abgeschätzt wird.

14. Verfahren nach einem der Ansprüche 11 bis 13, wobei die Lichtprojektion bei der Erzeugung der Bilddaten so eingestellt wird, dass eine Projektionsebene (13) der Lichtprojektion durch die beiden Pupillen (3R, 3L) des Benutzers (2) verläuft.

15. Computerprogrammprodukt, umfassend Programmteile, die bewirken, dass die Vorrichtung des Anspruchs 1 die Verfahrensschritte nach einem der Ansprüche 11 bis 14 ausführt.

## Claims

1. Device (10) for determining optical parameters of a user (2) with spectacles (4) arranged on the head of the user (2) in the position of use, having
- at least one projection device (12) which is designed and arranged to mark a partial region of the head of the user (2) and/or of the user's spectacles (4) with a light projection
- at least one image pick-up device (11) which is designed and arranged to generate image data of at least the marked partial area of the user's (2) head and/or the user's glasses (4), and
- a data processing device comprising
- user data determining means adapted to determine user data of the marked partial area of the head and/or the glasses (4) on the basis of the generated image data, the user data comprising location information in three-dimensional space of points of the partial area of the head and/or the glasses (4), and
- a parameter determining means adapted to determine optical parameters of the user (2) based on the user data;
wherein the projection device (12) is designed and arranged in such a way that individual points on the head and/or the glasses (4) of the user are specifically marked by the light projection in the image data,
in that in the image data at least one of the following user points is specifically marked by the light projection in the form of a line, a line intersection and/or a point:
- a pupil midpoint (PMR, PML),
- an outer temporal grasping point (ATR, ATL),
- an inner nasal grasping point (INR, INL),
- an inner focus point above the pupil (IOR, IOL) and/or
- an inner grasping point below the pupil (IUR, IUL).

2. Device according to claim 1, wherein said projection means (12) is calibrated relative to said image pickup means (11) and said user data determination means uses information about said calibration to determine said user data.

3. Device according to claim 1 or 2, wherein the projection means (12) has an adjustable projection direction (17).

4. Device according to any one of the preceding claims, wherein the light projection provided by the projection means (12) includes at least one projection plane (13) which at least partially projects a line (14) on the illuminated partial area of the user's head and/or glasses (4).

5. Device according to claim 4, wherein the projection device (12) is calibrated relative to the image pickup device (11) such that the optical axis (16) of the image pickup device (11) and the projection plane (13) intersect at a predetermined intersection angle of at least 10° and at most 70°.

6. Device according to any one of the preceding claims, wherein the light projection provided by the projection device (12) comprises at least two projection planes which intersect at a predetermined angle.

7. Device according to any one of the preceding claims, wherein the user data determining means is adapted and arranged to determine the user data from image data generated with a single shot of the image capturing means (11 ); or
wherein the user data determining means is adapted and arranged to determine the user data from two sets of image data from different shooting positions and to use the mark (14; 14') by the light projection to identify corresponding pixels in the two sets of image data.

8. Device according to any one of the preceding claims, wherein the projection means (12) provides the light projection in an invisible wavelength.

9. Device according to claim 8, comprising a preview output device (15) indicating to which part of the user's head and/or glasses (4) the invisible light projection is directed.

10. Device according to any of the preceding claims, wherein the device (10) is designed as a portable, mobile device.

11. Method for determining optical parameters of a user (2) with a pair of glasses (4) arranged on the head of the user (2) in the position of use, wherein:
- a partial area of the head of the user (2) and/or the glasses (4) of the user (2) is marked with a light projection,
- image data of at least the marked partial region of the head of the user (2) and/or the spectacles (4) of the user are generated,
- user data of the marked partial region of the head and/or of the glasses (4) are determined on the basis of the generated image data, the user data comprising location information in three-dimensional space of points of the partial region of the head and/or of the glasses (4),
- optical parameters of the user (2) are determined on the basis of the user data,
- individual points on the head and/or the glasses (4) of the user are specifically marked by the light projection in the image data in such a way that in the image data at least one of the following user points is specifically marked by the light projection in the form of a line, a line intersection and/or a point:
- a pupil midpoint (PMR, PML),
- an outer temporal grasping point (ATR, ATL),
- an inner nasal grasping point (INR, INL),
- an inner focus point above the pupil (IOR, IOL) and/or
- an inner grasping point below the pupil (IUR, IUL).

12. Method according to claim 11, wherein the user data is determined taking into account a calibration of the light projection relative to the positioning and orientation of an image capture device (11) for generating the image data.

13. Method according to claim 12, wherein a distance between the position of the image pickup device (11) and the position of the user (2) is estimated from the image data by triangulation taking into account the calibration.

14. Method according to any one of claims 11 to 13, wherein the light projection is adjusted when generating the image data such that a projection plane (13) of the light projection passes through the two pupils (3R, 3L) of the user (2).

15. Computer program product comprising program parts that cause the apparatus of claim 1 to perform the method steps of any one of claims 11 to 14.

## Revendications

1. Dispositif (10) pour déterminer des paramètres optiques d'un utilisateur (2) avec des lunettes (4) disposées sur la tête de l'utilisateur (2) en position d'utilisation, avec
- au moins un dispositif de projection (12), qui est conçu et disposé pour marquer une zone partielle de la tête de l'utilisateur (2) et/ou des lunettes (4) de l'utilisateur avec une projection de lumière,
- au moins un dispositif de prise de vue (11), qui est conçu et agencé pour générer des données d'image au moins de la zone partielle marquée de la tête de l'utilisateur (2) et/ou des lunettes (4) de l'utilisateur, et
- un dispositif de traitement de données comprenant
- un dispositif de détermination de données utilisateur qui est conçu pour déterminer, à l'aide des données d'image générées, des données utilisateur de la zone partielle marquée de la tête et/ou des lunettes (4), les données utilisateur comprenant des informations de lieu dans l'espace tridimensionnel de points de la zone partielle de la tête et/ou des lunettes (4) et
- un dispositif de détermination de paramètres, qui est conçu pour déterminer des paramètres optiques de l'utilisateur (2) à l'aide des données d'utilisateur ;
le dispositif de projection (12) étant conçu et disposé de telle sorte que la projection de lumière dans les données d'image marque de manière ciblée des points individuels sur la tête et/ou les lunettes (4) de l'utilisateur,
qu'au moins l'un des points suivants de l'utilisateur est marqué de manière ciblée dans les données d'image par la projection de lumière sous la forme d'une ligne, d'une intersection de lignes et/ou d'un point :
- un point central de la pupille (PMR, PML),
- un point de saisie temporal externe (ATR, ATL),
- un point de saisie nasal interne (INR, INL),
- un point de monture interne au-dessus de la pupille (IOR, IOL) et/ou
- un point de captage interne en dessous de la pupille (IUR, IUL).

2. Dispositif selon la revendication 1, dans lequel le moyen de projection (12) est calibré par rapport au moyen de capture d'image (11) et le moyen de détermination de données utilisateur utilise des informations sur ce calibrage pour déterminer les données utilisateur.

3. Dispositif selon la revendication 1 ou 2, dans lequel le dispositif de projection (12) présente une direction de projection réglable (17).

4. Dispositif selon l'une quelconque des revendications précédentes, dans lequel la projection de lumière fournie par le dispositif de projection (12) comprend au moins un plan de projection (13) qui projette au moins partiellement une ligne (14) sur la zone partielle éclairée de la tête et/ou des lunettes (4) de l'utilisateur.

5. Dispositif selon la revendication 4, dans lequel le dispositif de projection (12) est calibré par rapport au dispositif de prise de vue (11) de telle sorte que l'axe optique (16) du dispositif de prise de vue (11) et le plan de projection (13) se coupent selon un angle d'intersection prédéterminé d'au moins 10° et d'au plus 70°.

6. Dispositif selon l'une des revendications précédentes, dans lequel la projection de lumière fournie par le dispositif de projection (12) comprend au moins deux plans de projection qui se coupent selon un angle prédéterminé.

7. Dispositif selon l'une quelconque des revendications précédentes, dans lequel le moyen de détermination des données utilisateur est conçu et agencé pour déterminer les données utilisateur à partir des données d'image générées par une seule prise de vue du moyen de prise de vue (11) ; ou
dans lequel le dispositif de détermination des données utilisateur est conçu et agencé pour déterminer les données utilisateur à partir de deux ensembles de données d'image provenant de positions de prise de vue différentes et pour utiliser le marquage (14 ; 14') par la projection de lumière pour identifier des points d'image correspondants dans les deux ensembles de données d'image.

8. Dispositif selon l'une quelconque des revendications précédentes, dans lequel le dispositif de projection (12) fournit la projection de lumière dans une longueur d'onde invisible.

9. Dispositif selon la revendication 8, comprenant un dispositif de sortie d'aperçu (15) indiquant sur quelle partie de la tête et/ou des lunettes (4) de l'utilisateur la projection de lumière invisible est orientée.

10. Dispositif selon l'une des revendications précédentes, dans lequel le dispositif (10) est conçu comme un dispositif portable et mobile.

11. Procédé de détermination de paramètres optiques d'un utilisateur (2) avec des lunettes (4) disposées sur la tête de l'utilisateur (2) en position d'utilisation, dans lequel :
- une zone partielle de la tête de l'utilisateur (2) et/ou des lunettes (4) de l'utilisateur (2) est marquée par une projection de lumière,
- des données d'image d'au moins la zone partielle marquée de la tête de l'utilisateur (2) et/ou des lunettes (4) de l'utilisateur sont générées,
- à l'aide des données d'image générées, on détermine des données d'utilisateur de la zone partielle marquée de la tête et/ou des lunettes (4), les données d'utilisateur comprenant des informations de lieu dans l'espace tridimensionnel de points de la zone partielle de la tête et/ou des lunettes (4),
- des paramètres optiques de l'utilisateur (2) sont déterminés à l'aide des données utilisateur,
- la projection de lumière dans les données d'image marque de manière ciblée des points individuels sur la tête et/ou les lunettes (4) de l'utilisateur de telle sorte que, dans les données d'image, au moins l'un des points d'utilisateur suivants est marqué de manière ciblée par la projection de lumière sous la forme d'une ligne, d'une intersection de lignes et/ou d'un point :
- un point central de la pupille (PMR, PML),
- un point de saisie temporal externe (ATR, ATL),
- un point de saisie nasal interne (INR, INL),
- un point de monture interne au-dessus de la pupille (IOR, IOL) et/ou
- un point de monture interne en dessous de la pupille (IUR, IUL).

12. Procédé selon la revendication 11, dans lequel les données utilisateur sont déterminées en tenant compte d'un étalonnage de la projection de lumière par rapport au positionnement et à l'orientation d'un dispositif de prise de vue (11) pour générer les données image.

13. Procédé selon la revendication 12, dans lequel une distance entre la position du dispositif de prise de vue (11) et la position de l'utilisateur (2) est estimée à partir des données d'image par triangulation en tenant compte de l'étalonnage.

14. Procédé selon l'une des revendications 11 à 13, dans lequel la projection de lumière est ajustée lors de la génération des données d'image de sorte qu'un plan de projection (13) de la projection de lumière passe par les deux pupilles (3R, 3L) de l'utilisateur (2).

15. Produit de programme d'ordinateur comprenant des parties de programme qui font que le dispositif de la revendication 1 exécute les étapes de procédé selon l'une des revendications 11 à 14.
